(19) **Europäisches Patentamt**

**European Patent Office**

**Office européen des brevets**

(11) Veröffentlichungsnummer: **0 337 264**
**A2**

(12) **EUROPÄISCHE PATENTANMELDUNG**

(21) Anmeldenummer: **89105927.1**

(22) Anmeldetag: **05.04.89**

(51) Int. Cl.⁴: **C12P 21/00**

(30) Priorität: **09.04.88 DE 3811921**

(43) Veröffentlichungstag der Anmeldung:
**18.10.89 Patentblatt 89/42**

(84) Benannte Vertragsstaaten:
**AT BE CH DE ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Habermann, Paul, Dr.**
**Rossertstrasse 35**
**D-6239 Eppstein/Taunus(DE)**

(54) **Verfahren zur Herstellung von Proteinen, die N-terminal mit Prolin beginnen.**

(57) Das Enzym Aminopeptidase-P eignet sich zur Herstellung von Proteinen, die N-terminal mit Prolin beginnen, aus gentechnisch erhaltenen Ausgangsmaterialien, die am N-terminalen Ende - insbesondere durch Methionin -verlängert sind.

EP 0 337 264 A2

## Verfahren zur Herstellung von Proteinen, die N-terminal mit Prolin beginnen

Es ist bereits bekannt, auf gentechnischem Wege Proteine herzustellen, die N-terminal mit Prolin beginnen. Hierzu wird zunächst ein längeres Protein exprimiert, in dem vor dem Prolin, das den Anfang des gewünschten Proteins darstellt, Asparaginsäure oder eine kurze Aminosäuresequenz, die C-terminal Asparaginsäure enthält, steht. Dieses Zwischenprodukt wird dann proteolytisch gespalten, wobei das gewünschte Protein mit dem Prolin-Anfang freigesetzt wird. Solche Verfahren sind in den europäischen Patentanmeldungen mit den Veröffentlichungsnummern (EP-A) 0 219 781, 0 227 938 und 0 228 018 beschrieben.

Dieses bekannte Verfahren ist selbstverständlich nur dann anwendbar, wenn das freigesetzte Protein hinreichend säurestabil ist. Aber auch bei relativ säurestabilen Proteinen besteht die Gefahr, daß Nebenreaktionen auftreten, wodurch einerseits unmittelbar die Ausbeute verringert wird und andererseits eine weitere Ausbeutereduzierung durch die schwierige Abtrennung von den unerwünschten Nebenprodukten eintritt.

Es wurde nun ein Verfahren gefunden, das den vorstehend genannten Beschränkungen nicht unterworfen ist und auch die geschilderten Nachteile nicht aufweist. Erfindungsgemäß werden Proteine, die N-terminal mit Prolin beginnen, dadurch hergestellt, daß ein gentechnisch gewonnenes Vorprodukt mit einer kurzen N-terminalen Verlängerung der enzymatischen Spaltung mit Aminopeptidase-P unterworfen wird.

Die kurze N-terminale Verlängerung im Vorprodukt besteht insbesondere aus einem Methioninrest, wie er bei der normalen Direktexpression von Proteinen aus Bakterien auftritt. Bevorzugte Ausgangsmaterialien sind aber auch Proteingemische aus Proteinen, die nur teilweise am N-Ende einen Methioninrest tragen, wie sie bei der Aufarbeitung von direkt exprimierten Produkten auftreten können. Solche Proteingemische konnten mit den bisher bekannten Verfahren nur sehr schwer aufgetrennt werden.

Schwierigkeiten mit der Abtrennung der Aminopeptidase-P vom gewünschten Protein ergeben sich wegen der Größe des Enzyms nicht. Außerdem ist es möglich, das zur Spaltung verwendete Enzym in an sich bekannter Weise zu immobilisieren, beispielsweise nach den Verfahren der europäischen Patentschriften (EP-B) 0 141 223 und 0 141 224 sowie der darin genannten Literatur.

Die Aminopeptidase-P ist aus A. Yaron et al., Biochemical and Biophysical Research Communications 32 (1968) 658-663, bekannt, wo auch beschrieben ist, daß sie Peptide N-terminal bis zu einem endständigen Prolin angreift. Als abgetrennte Aminosäuren sind jedoch nur die neutralen Aminosäuren Glycin, Alanin, Valin, Isoleucin und Prolin, sofern dieses von einem weiteren Prolin gefolgt wird, sowie die basische Aminosäure Arginin genannt.

Die Aminopeptidase-P ist nach dem von Yaron et al. beschriebenen Verfahren leicht isolierbar. Das Molgewicht von etwa $2 \cdot 10^5$ liegt in einer Größenordnung, die eine Abtrennung von den gewünschten Proteinen auch ohne Immobilisierung erlaubt. Dieses Enzym eignet sich somit hervorragend zur Gewinnung von Proteinen, die N-terminal mit Prolin beginnen, aus gentechnisch gewonnenen Vorprodukten, insbesondere aus Produkten der direkten Expression aus Bakterien, da dieses Enzym auch Methionin rasch und quantitativ abspaltet.

In den folgenden Beispielen wird die Erfindung näher erläutert. Prozentangaben beziehen sich auf das Gewicht.

**Beispiel 1**

Die Isolierung und Reinigung der Aminopeptidase-P erfolgt nach Yaron et al., a.a.O., mit den folgenden Modifikationen:
E. coli B-Zellen werden in 50 mM Natriumphosphat-Puffer aufgeschlossen, zentrifugiert und der rohe Zellextrakt 15 Minuten bei 50°C inkubiert. Das Gemisch wird erneut zentrifugiert und eine Ammoniumsulfatfällung bei 45 % Sättigung durchgeführt. Nach Zentrifugation findet man das Enzym im Überstand. Die weitere Reinigung erfolgt entsprechend dem bekannten Verfahren.

**Beispiel 2**

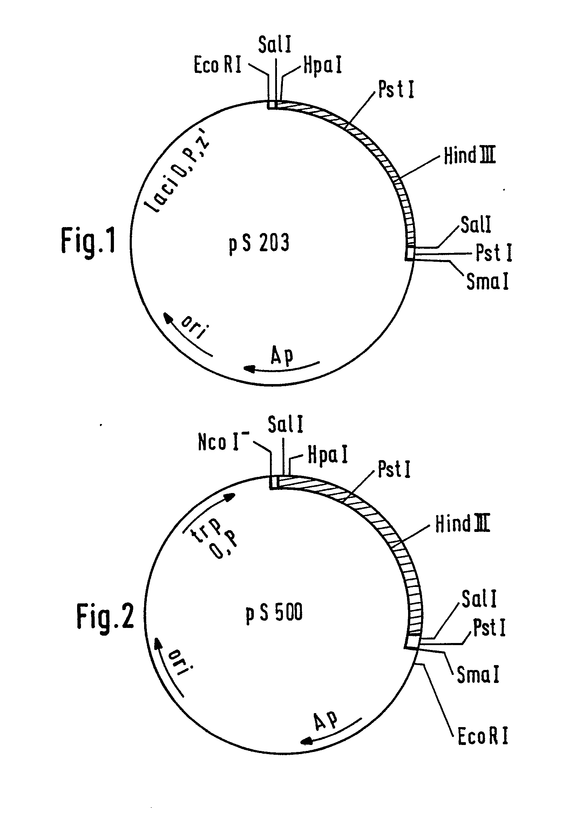

Als Ausgangsmaterialien für Plasmidkonstruktionen dienen die Plasmide pS203 und pPH160 aus der EP-A 0 228 018.
Das Plasmid pS203 findet sich in der EP-A 0 228 018 in Figur 10a als (78). Es enthält das synthetische Gen für den humanen Granulozyten-Makrophagen-"colony stimulating"-Faktor (GM-CSF). Dieses Gen enthält eine Vielzahl von Schnittstellen für Restriktionsenzyme, die in den Tabellen auf den S. 9 bis 11 der EP-A 0 228 018 wiedergegeben sind. Die für die folgenden Konstruktionen maßgeblichen Schnittstellen in pS203 sind in der vorliegenden Figur 1 eingezeichnet.

Das Plasmid pS203 wird mit SmaI und partiell mit SalI verdaut und das etwa 380 bp große Fragment isoliert, das das synthetische Gen für GM-CSF ab Aminosäure 9/10 enthält. Dieses Fragment wird in den handelsüblichen Vektor pUC18 eingesetzt, der mit SalI und SmaI geöffnet wurde. Kompetente E. coli 79/02-Zellen werden mit dem Ligationsgemisch inkubiert und das Transformationsgemisch auf IPTG-Xgal-Platten ausgestrichen, die 20 µg/ml Ampicillin enthalten. Nach Inkubation über Nacht werden weiße Kolonien isoliert und die Plasmid-DNA gewonnen. Die Plasmid-DNA wird durch Restriktionsanalyse charakterisiert. Die Plasmide mit dem erwarteten Restriktionsmuster erhalten die Bezeichnung pS499.

Das Plasmid pS499 wird mit EcoRI und partiell mit SalI verdaut und das Fragment isoliert, das das synthetische GM-CSF-Gen enthält. Dieses Fragment soll nun in das Expressionsplasmid pPH160 eingesetzt werden. Hierzu wird das Oligonukleotid (1)

```
                    1                              8

          Met Pro Ala Pro Ala Arg Ser Pro Ser Pro
    5' - C ATG CCG GCG CCG GCC CGA TCG CCG TCT CCG        - 3'
              GGC CGC GGC CGG GCT AGC GGC AGA GGC AGC T
                                                   (SalI)
```

(1)

synthetisiert und mit dem isolierten Fragment und dem mit den Enzymen NcoI und EcoRI geöffneten Plasmid pPH160 ligiert. Hierdurch wird die NcoI-Schnittstelle zerstört (in der Figur 2: NcoI⁻). Mit dem Ligationsgemisch werden kompetente E. coli MM294-Zellen transformiert und die Plasmide durch Restriktions- und DNA-Sequenzanalyse identifiziert. Plasmide mit dem erwarteten Restriktionsmuster erhalten die Bezeichnung pS500 (Figur 2). Dieses Plasmid kodiert für ein GM-CSF-Derivat, das N-terminal um Met-Pro verlängert ist.

**Beispiel 3**

Im folgenden werden analoge Plasmidkonstruktionen beschrieben, die die Direktexpression von GM-CSF-Derivaten erlauben, die aus der EP-A 0 228 018 bekannt sind.

Für diese Konstruktionen wird von der HpaI-Schnittstelle im Plasmid pS203 bzw. pS499 Gebrauch gemacht.

Analog Beispiel 2 wird pS499 mit EcoRI und HpaI verdaut und das Fragment mit dem synthetischen GM-CSF-Gen isoliert. Anstelle des Oligonukleotids (1) wird nun aber das Oligonukleotid (2)

```
                  2                              10

          Met Pro Ala Arg Ser Pro Ser Pro Ser Thr Gln
    5' - C ATG CCG GCC CGA TCG CCG TCT CCG TCG ACC CAG -
              GGC CGG GCT AGC GGC AGA GGC AGC TGG GTC
                                                         (2)

                  16

          Pro Trp Glu His Val
          CCC TGG GAA CAC GTT - 3'
          GGG ACC CTT GTG CAA
                      (HpaI)
```

3

eingefügt. Man erhält so das Plasmid pS502.

Ersetzt man das Oligonukleotid (2) durch das Oligonukleotid (3)

```
              6                  10                        16
        Met Pro Ser Pro Ser Thr Gln Pro Trp Glu His Val
  5' - C ATG CCG TCT CCC TCG ACC CAG CCC TGG GAA CAC GTT - 3'
        GGC AGA GGG AGC TGG GTC GGG ACC CTT GTG CAA
                                                        (HpaI)
                    (3)
```

so erhält man das Plasmid pS503.

Analog erhält man durch Einfügung des Oligonukleotids (4)

```
          8       10                        16
      Met Pro Ser Thr Gln Pro Trp Glu His Val
5' - C ATG CCG TCG ACC CAG CCC TGG GAA CAC GTT - 3'     (4)
      GGC AGC TGG GTC GGG ACC CTT GTG CAA
                                            (HpaI)
```

das Plasmid pS504.

Entsprechend erhält man durch Einfügen des Oligonukleotids (5)

```
              12              16
          Met Pro Trp Glu His Val
    5' - C ATG CCG TGG GAA CAC GTT - 3'            (5)
          GGC ACC CTT GTG CAA
                        (HpaI)
```

das Plasmid pS505.

Zur Verdeutlichung ist in den Oligonukleotiden die Aminosäurenumerierung für GM-CSF angegeben.

Die Expression aller GM-CSF-Derivate erfolgt nach Beispiel 1 der EP-A 0 228 018. Das GM-CSF-Derivat liegt in der Zelle je nach Fermentationsbedingungen gelöst oder als unlöslicher Einschlußkörper vor.

Im letzteren Fall läßt sich das Protein nach Zellaufschluß durch Zentrifugation im Sediment anreichern. Das Sediment wird mit Wasser gewaschen und anschließend in Desoxycholatlösung resuspendiert. Die Suspension wird zentrifugiert, der Rückstand erneut mit Wasser gewaschen und in 6 M Harnstoff-haltigem 50 mM Tris-Puffer (pH 8) aufgelöst (50 mg Protein pro 4 ml Wasser).

Zur Sulfonatbildung des Proteins werden zu dem Überstand bzw. zu der Lösung ca. 15 mg Natriumsulfit zugegeben und das Gemisch etwa 120 Minuten bei Raumtemperatur stehen gelassen. Nach Verdünnen auf 10 ml wird ein pH von 4,7 eingestellt und das Gemisch über Nacht stehen gelassen. Der Niederschlag wird abzentrifugiert, das Sediment mit Wasser gewaschen und erneut in einem Puffer (5 M Harnstoff, 50 mM NaCl, 50 mM Tris) gelöst. Diese Lösung wird auf eine Anionen-Austauschersäule gegeben, die mit ®FRACTOGEL DEAE TSK-Material (45 bis 90 μm; Merck) gefüllt und gegen 6 M Harnstoff, 50 mM Tris, 50 mM NaCl, 0,1 % (w/v) Tensid (®TWEEN), 2 mM Ethylendiamin (pH 8,7) äquilibriert wurde. Das GM-CSF-S-Sulfonat eluiert im Bereich 140 bis 210 mM NaCl.

Die GM-CSF-S-Sulfonat enthaltenden Fraktionen werden vereinigt, auf eine Proteinkonzentration von $10^{-5}$ Mol eingestellt und anschließend gegen einen Mn-Citrat-Reaktionspuffer (pH 8,6; $Mn^{2+}$-Konzentration $3.10^{-5}$ M) dialysiert. Dann wird das GM-CSF-S-Sulfonat auf eine Konzentration von $10^{-5}$ Mol eingestellt und mit 0,5 bis 1 μg/ml Aminopeptidase-P eine Stunde bei 37-40° C umgesetzt. Die Reaktionspartner werden

4

anschließend durch Gelchromatographie (®SEPHADEX G 75-Säule; Pharmacia) voneinander getrennt. Das GM-CSF wird dann durch Verdünnen in Gegenwart von Sauerstoff in einem 50 mM Glycin-Puffer renaturiert.

Das renaturierte Produkt wird konzentriert und zur Proteinsequenzanalyse über HPLC (Säule: Waters $C_{18}$, 250•4,6 mm; Puffer A: 0,1 % Trifluoressigsäure, Puffer B: 80 % Acetonitril, 0,1 % Trifluoressigsäure) gereinigt. Ein Gradient von 35 % B in 30 Minuten zu 75 % B mit einer Flußrate von 1,5 ml wird eingestellt. Die GM-CSF-haltigen Fraktionen werden der automatisierten Proteinsequenzanalyse unterworfen. Es zeigt sich, daß das Methionin praktisch quantitativ abgespalten wurde. Demgegenüber enthielten nicht mit Aminopeptidase-P behandelte Produkte zu > 50 % Methionin als N-terminale Aminosäure.

## Ansprüche

1. Verfahren zur Herstellung von Proteinen, die N-terminal mit Prolin beginnen, dadurch gekennzeichnet, daß ein gentechnisch gewonnenes Vorprodukt mit einer kurzen N-terminalen Verlängerung der enzymatischen Spaltung mit Aminopeptidase-P unterworfen wird.

2. Verfahren nach Anspruch 1, dadurch gekennzeichnet, daß die kurze N-terminale Verlängerung aus einem Methioninrest besteht.

3. Verfahren nach Anspruch 1 oder 2, dadurch gekennzeichnet, daß als Ausgangsmaterial ein Gemisch aus Proteinen dient, die nur teilweise am N-Ende einen Methioninrest tragen.

4. Verfahren nach einem oder mehreren der vorhergehenden Ansprüche, dadurch gekennzeichnet, daß ein N-terminal verlängertes, mit Prolin beginnendes Derivat des humanen Granulozyten-Makrophagen-"colony stimulating"-Faktor eingesetzt wird.

Fig.1

EcoRI  SalI  HpaI  PstI

HindIII

SalI
PstI
SmaI

laciO,P,z'

pS 203

ori

Ap

Fig.2

NcoI⁻  SalI  HpaI  PstI

HindIII

SalI
PstI
SmaI

EcoRI

trp
O,P

pS 500

ori

Ap